# NEW EUROPEAN PATENT SPECIFICATION

(11) **EP 0 794 248 B2**
(45) Date of publication and mention of the opposition decision: **07.04.2010**
(45) Mention of the grant of the patent: 10.10.2001
(21) Application number: 97200679.5
(22) Date of filing: 10.03.1997
(51) Int. Cl.: A21D 8/04

(54) **Improved method of dosing cream yeast**
Dosierung von Hefekreme
Dosage de crème de levure

(30) Priority: 08.03.1996 EP 96200595
(43) Date of publication of application: 10.09.1997
(73) Proprietor: Lesaffre et Compagnie, 75001 Paris (FR)
(72) Inventor: Jellema, Sjoerd Hette, 3263 NE Oud-Beijerland (NL); van Lammeren, Gijsbertus Petrus Antonius Paulina, 4624 EJ Bergen op Zoom (NL)
(74) Representative: Hirsch & Associés

(56) References cited:
- EP-A- 0 123 873
- WO-A-94/18111
- WO-A-95/15520
- DE-A- 2 219 041
- DE-A- 3 633 042
- DE-A- 4 237 904

## Description

The present invention relates to the dosing of cream yeast.

The industrial production of baker's yeast began in Europe and North America more than 100 years ago. However, yeast has been used for thousands of years as the leavening agent of dough in bread making. During the last century, the production process of baker's yeast was developed to a fed-batch process with high yields. This has been the basis for many other fermentation processes.

The manufacture of baker's yeast starts with a small sample of a pure culture. This sample is used to inoculate the first of a series of fermentors of successively increasing size. The first few fermentation stages are mildly aerated batch fermentations. In these stages, the conditions are such that ethanol is formed. Only the last two (or sometimes three) fermentation stages are performed using full aeration with incremental feeding of molasses. These fed-batch fermentations are carried out in fermentors of 100 m³ or more net volume. The fermentation time is typically in the range of 12-20 hours, in which some 20,000-30,000 kg of fresh yeast is produced. After the feeding of substrates has stopped, aeration is usually continued at a reduced level for half an hour or so to let the yeast cells attain maturity and uniformity. Further processing includes separation of the yeast from the broth by centrifugation, washing and optionally diluting which results in cream yeast (10-24wt% dry matter content).

Cream yeast is also known to be a valuable yeast product for use in baked foods (see for example Dutch patent application No. 259948). However, the production of cream yeast for direct use in baking processes has never been a commercial success. One of the main problems is the phase separation that occurs in the cream yeast tanks during transport and storage. It has been found that a liquid yeast suspension shows a perceptible yeast concentration gradient in the vertical direction within 6 hours. The cause of this is that yeast cells have a higher density than water and gradually sink to the bottom (phase separation). This leads to a 'clayish' suspension being formed on the bottom of the storage container, which is difficult to resuspend with the remaining liquid.

For commercial yeast production in which cream yeast is processed into compressed yeast, it is known to maintain the cream yeast in a well-mixed condition by the use of a stirrer. This solution, the use of a stirrer during transport of storage of cream yeast for direct use in baking processes, has been suggested in Dutch patent application no. 259948.

An alternative known way of maintaining cream yeast in a homogeneous state is to use a pump to withdraw part of the cream yeast from its container and subsequently to reintroduce the cream yeast into the container in such a way that the cream yeast remains in a well-mixed state.

These existing solutions to the problem of how to keep cream yeast in an homogeneous state require complicated apparatus for the yeast producer, the transporter and the baker. Moreover, because yeast is easily infected, regular cleaning of this apparatus is necessary.

Published European Patent Application No. 461725 discloses that by the addition of a small amount of gum cream yeast can be kept in an homogeneous state during transport as well as during storage. In this way stabilised cream yeast is obtained. By stabilized cream yeast is meant a cream yeast which is kept in a homogeneous state by an additive without the use of mechanical means such as a stirrer. In general, the cream yeast is stabilized by a viscosity regulating agent such as xanthane gum.

Stabilization of cream yeast as described in EP-A-461725 means less expensive containers for transport and storage are necessary and an increase in temperature of the yeast cream due to recirculation or stirring is avoided. Furthermore, more freedom is obtained in choosing the volume of the containers. Either large containers or small containers with a volume as little as 1 or 2 litres can be used. Small volumes are especially suitable for the preparation of home-baked goods.

The use of cream yeast makes automation of the addition of a chosen quantity of yeast to a dough or to dough ingredients possible. With the help of e.g. a pump and a mass or volume meter and a dosing valve, a fixed amount of liquid cream yeast can be added. However, in practice, it is found that the yeast in the container tends to form small amounts of gas which expand between the pump and the dosing valve. A possible explanation for this phenomenon might be the pressure relief after the pump.

A consequence of the formation of gas is that when no cream yeast is dosed, a two phase stream occurs. The gas will rise and will drive away liquid cream yeast. When this effect occurs between a mass or volume meter and the dosing valve, the subsequent dosing of cream yeast will at least partly consist of gas instead of yeast and a too small amount of yeast will be dosed. Because this effect is influenced by many factors, e.g. time and temperature of the liquid yeast, the amount of gas formation is not constant and not predictable. Furthermore, some mass and volume meters become disturbed by gas in the liquid flow. Therefore, all the benefits of the liquid stabilised cream yeast are at least partly set aside by the dosing problems.

DE-A-4237904 discloses a process and device for accurate dosing of yeast cells from a yeast suspension independently of the viscosity of the suspension. The process involves variation of the suspension volume comprised between the pressurized regions (the container) and the release region (the dosage valve) by introduction of a secondary liquid stream, resulting in a modulation of the viscosity of the original suspension up to a desired values.

We have now found that if at least part of the connecting means between the container and the dosing valve is kept at a pressure above atmospheric pressure, the formation of an inhomogeneous medium is substantially prevented and accurate dosing becomes possible. In particular, if a mass or volume meter is employed, at least the cream yeast present in the pipe(s) between the mass or volume meter and the dosage valve is kept under an overpressure.

Although the use of pressurized conduit lines in dosing units is known from WO-A-95/15520 and WO-A-94/18111, none of these documents envisage the technical problem of the present application.

Thus, in one aspect, the present invention provides a method of dosing stabilized cream yeast, employing a dosing unit wherein cream yeast is transported from a storage container via a connecting means to a dosage valve for release, characterised in that the cream yeast is kept under a pressure above atmospheric for at least a proportion of the time when in said connecting means, and the dosage valve, such that the accuracy of yeast dosing is not substantially affected by gas formation and wherein a pump, situated between the container in which the cream yeast is stored and a mass or volume meter, is employed to pump cream yeast through said connecting means and maintain the cream yeast under a pressure above atmospheric during transport from the pump to the dosage valve. It is also possible to create an overpressure in the container as well, in which case the cream yeast is kept under an overpressure until the cream yeast is dosed. In general, an overpressure of 0.2 to 10 bar, preferably 0.5 to 6 bar is used.

By means of methods according to the invention, it is possible to prevent a substantial amount of CO₂ formed by the yeast being separately present in the mass or volume meter, if present, or during the dosing from the dosing valve. More accurate dosing is thus possible.

The stabilized cream yeast can be dosed using a dosing unit comprising
- a storage container for cream yeast;
- a dosing means to dose the cream yeast;
- a connecting means, connecting the storage container and the dosing means,
- means to determine the amount of cream yeast to be dosed;
- a pump, located between the storage container and the means to determine the amount of cream yeast to be dosed
whereby the cream yeast to be dosed is kept under a pressure above atmospheric least in part of the connecting means whereby said part is directly connected to the dosing means.

It will be appreciated that in all embodiments, an amount of cream yeast will be present in the pipe line(s) between the container and the dosing valve, which should be seen as 'dead space' in the dosing system and which should, if necessary, be taken into account when the cream yeast is dosed. The dosing valve is preferably a spring closed sanitary valve. For the control of the dosing valve on basis of signals from the mass or volume meter or the weighing of the container, computer apparatus is commercially available.

It will be appreciated that the chosen flow through the system depends on the amount to be dosed and the desired accuracy. It will also be appreciated that the skilled in the art will be able to dose a desired amount of cream yeast by hand or automatically with the help of apparatus as disclosed herein.

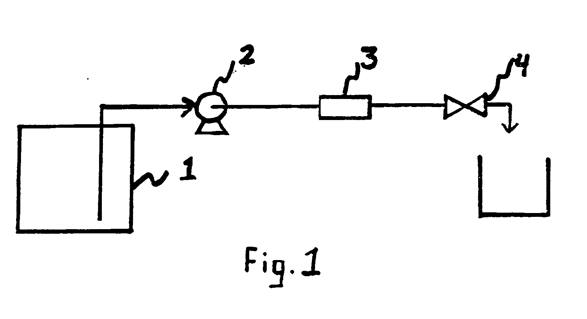

Figure 1 schematically shows an embodiment of apparatus for dosing cream yeast in accordance with the invention.

Container (1) storing cream yeast is shown. In practice, more than one container can be used. When the container is empty, a second container can be connected without delay. Subsequently the empty container can be replaced by a refilled one. Another possibility is that the container is re-filled after a certain period of time (e.g. once a week) and in this case the container is not moved from the yeast producing factory or bakery. The yeast container is preferably a plastic or stainless steel container.

The cream yeast is pumped via pump (2) and the mass or volume meter (3) to the dosing valve (4). The pump may be an air or electric driven pump to pump the yeast from the container to the dosing point and, if necessary, to pressurize the yeast.

We have found advantageously for measuring the amount of yeast dosed, a mass meter can be used. Several mass meters are commercially available which can be used without any adjustments. The flow or mass flow meter is preferably a mass flow meter operating according to the corriolise measuring principle.

As indicated above, the cream yeast dosed via valve (4) can alternatively be determined by weighing the container of cream yeast. The loss of weight corresponds with the amount of yeast leaving through valve (4).

## Claims

1. A method of dosing stabilized cream yeast employing a dosing unit wherein cream yeast is transported from a storage container via a connecting means to a dosage valve for release, **characterised in that** the cream yeast is kept under a pressure above atmospheric for at least a proportion of the time when in said connecting means, and the dosage valve, such that the accuracy of yeast dosing is not substantially affected by gas formation and wherein a pump, situated between the storage container and a mass or volume meter, is employed to pump cream yeast through said connecting means and maintain the cream yeast under a pressure above atmospheric during transport from the pump to the dosage valve.

2. A method as claimed in claim 1 wherein the pressure is raised by 0.2 to 10 bar above atmospheric.

3. A method as claimed in any one of claims 1 or 2 wherein the pressure is raised by 0.5 to 6 bar above atmospheric.

## Patentansprüche

1. Verfahren zum Dosieren von stabilisierte Cremehefe unter Einsatz einer Dosiereinheit, wobei Cremehefe von einem Lagerbehälter über ein Verbindungselement zu einem Dosierventil für die Abgabe transportiert wird, **dadurch gekennzeichnet, dass** die Cremehefe während mindestens eines Teils der Zeit, in der sie sich in dem genannten Verbindungselement und in dem Dosierventil befindet, derart unter einem Druck oberhalb des Atmosphärendrucks gehalten wird, dass die Genauigkeit der Hefedosierung durch eine Gasbildung nicht wesentlich beeinträchtigt wird und worin eine zwischen dem Lagerbehälter und dem Massen- oder Volummeter angeordnete Pumpe eingesetzt wird, um die Cremehefe durch das genannte Verbindungselement zu pumpen und während des Transports von der Pumpe zu dem Dosierventil unter einem Druck oberhalb des Atmosphärendrucks zu halten.

2. Verfahren nach Anspruch 1 oder 2, worin der Druck um 0,2 bis 10 bar über des Atmosphärendrucks erhöht wird.

3. Verfahren nach Anspruch 1, worin der Druck um 0,5 bis 6 bar über des Atmosphärendrucks erhöht wird.

## Revendications

1. Procédé de dosage de crème de levure stabilisée utilisant une unité de dosage dans laquelle la crème de levure est transportée d'un conteneur de stockage, via un moyen de connexion, à une valve de dosage pour être libérée, **caractérisé en ce que** la crème de levure est conservée sous une pression supérieure à la pression atmosphérique pendant au moins une proportion de temps, quand elle est dans ledit moyen de connexion, et la valve de dosage, telle que la précision de dosage de levure n'est pratiquement pas affectée par la formation de gaz et dans lequel une pompe, située entre le conteneur de dosage et le doseur de masse ou de volume, est utilisée pour pomper la crème de levure à travers ledit moyen de connexion et maintenir la crème de levure sous une pression supérieure à la pression atmosphérique durant le transport de la pompe à la valve de dosage.

2. Procédé tel que revendiqué dans la revendication 1 dans lequel la pression est augmentée de 0,2 à 10 bars au dessus de la pression atmosphérique.

3. Procédé tel que revendiqué dans une quelconque des revendications 1 ou 2 dans lequel la pression est augmentée de 0,5 à 6 bars au dessus de la pression atmosphérique.
